# EUROPEAN PATENT APPLICATION

(11) **EP 2 415 780 A1**
(43) Date of publication of application: **08.02.2012**
(21) Application number: 10758012.8
(22) Date of filing: 17.03.2010
(51) Int. Cl.: C07K 7/00, C07K 7/08, C07K 14/00, A61K 38/04, A61K 38/16, A61P 35/00, A61P 37/04

(54) **SYNTHETIC PEPTIDE AND USES THEREOF**

(30) Priority: 30.03.2009 CN 200910131430
(71) Applicant: Hebei Yuansen Pharmaceutical Co., Ltd., Hebei 072750 (CN); Yuansentai Biotech (Tianjin) Inc., Tianjin 300457 (CN)
(72) Inventor: HAN, Su, Beijing 100105 (CN); TIAN, Deqing, Beijing 100105 (CN); YUAN, Shoujun, Beijing 100105 (CN); JIAO, Shunchang, Beijing 100105 (CN)
(74) Representative: Hryszkiewicz, Danuta
(86) International application number: PCT/CN2010/071096
(87) International publication number: WO 2010/111910

(57) **Abstract**

The present invention discloses a 4-copy branched peptide and uses thereof. The structure formula of said branched peptide is (X_{A}-X_{B}-X_{C}-X_{D}-X₁)₄>(K-X₂)₂>K-X₃. Said branched peptide shows significant inhibition of tumor growth and immunostimulating effect in animal experiments, and has the potential to be developed into clinical anti-tumor drugs and immunostimulants.

## Description

This application claims the benefit of Chinese Patent Application No. 200910131430.6 filed Mar. 30, 2009.

The invention relates to a synthetic peptide and uses thereof, and more particularly, to a 4-copy branched peptide capable of inhibiting tumor growth and enhancing immunity as well as uses thereof.

Genetic genes of organisms are stored in polynucleotide chains and encode proteins with biological functions. Proteins vary in organisms and exhibit various biological functions to sustain living activities. Although there are different types of proteins, they are basically composed of twenty natural amino acids. The difference of proteins lies in the amino acid composition and sequence. Generally, a molecule consisting of over 50 amino acids is named as a protein; a peptide chain consisting of over 10 amino acids is named as a polypeptide; and a peptide chain consisting of less than 10 amino acids is named as an oligopeptide. Up to now, the smallest functional peptide discovered consists of only two amino acids. Small functional peptides consisting of more than four amino acids are very common.

The accomplishment of human genome project and the start-up of human proteome project have made more and more protein functional fragments discovered and applied to biomedicine. Functional fragments of proteins are basically linear chain polypeptide fragments with specific biological functions. They are generally composed of two to dozens of amino acids and can be artificially synthesized. For example, oxytocin, thymosin α1, and thymopeptide-5 are synthesized polypeptide drugs which have been widely applied in clinic. Other polypeptide drugs, for example, octreotide used for treatment of gastrointestinal bleeding and acromegaly and hirudin peptide with anticoagulation function, are prepared using artificial mutants based on natural peptide chains. Functional fragments of proteins can be screened to contain dozens of or only two amino acids, which lay a solid foundation for artificial synthesis of functional fragments of peptides as well as application thereof.

Original bioactivities of proteins, polypeptides, or oligopeptides can be changed unpredictably due to deletion, insertion, or replacement of a single amino acid, blockage of the amino terminal (N terminal) or the carboxyl terminal (C terminal), or modification of an amino acid in the middle or at free end of a sequence by chemical groups. Design, screening, and discovery of peptides with novel functions or searching for efficient peptide are a key step for drug development.

Tuftsin, found by American scientists (Life Science, 1981, 26(10): 1081-1091), is an active 4-peptide Thr-Lys-Pro-Arg or TKPR derived from spleen. Studies show that Tuftsin can increase acinus lienis inside spleen, activate the growth of germinal center, strengthen chemotaxis, dissociation, swallowing, and cell toxicant generation of granulocytes, monocytes, macrophages, and natural killer cells, and improve cellcular immunity of lymphatic system. Tuftsin can promote both MHC unrestrictive function of mononuclear phagocytes and the restrictive antigen presentation, and improve cell toxicant function.

The invention relates to a peptide comprising a 4-copy TKPR fragment for effectively improving the immunity and anti-tumor activity, and to the development of an anti-tumor drug and immunity enhancer using the peptide for clinical applications.

One objective of the invention is to provide a 4-copy branched peptide and the uses thereof as an anti-tumor drug and immunity enhancer. Technical problems to be solved are to maintain and improve biological activities of functional oligopeptide of the 4-copy branched peptide.

The objective of the invention is achieved using the following technical scheme. The invention provides a 4-copy branched peptide comprising 4 active oligopeptide fragments linked using lysine comprising two free activated amino groups for amino acid addition condensation reactions, the 4-copy branched peptide having a formula of (X_{A}-X_{B}-X_{C}-X_{D}-X₁)₄>(K-X₂)₂>K-X₃, 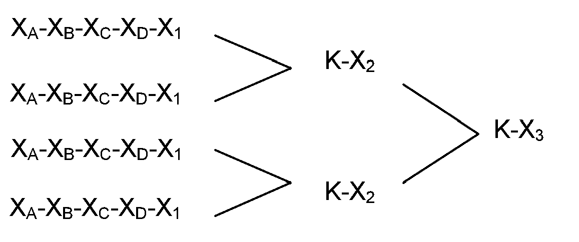 wherein >K- is lysine comprising two free activated amino groups for amino acid addition condensation reactions; X_{A} and X_{c} separately represent an uncharged polar amino acid comprising serine (Ser, S), threonine (Thr, T), cysteine (Cys, C), praline (Pro, P), glutamine (Gln, Q), and asparagine (Asn, N); X_{B} and X_{D} separately represent an alkaline amino acid comprising histidine (His, H), lysine (Lys, K), and arginine (Arg, R); X₁ and X₂ separately represents an amino acid sequence comprising between 0 and 5 random amino acids, and X₁ and X₂ are the same, different, or absent; and X₃ is a sequence comprising between 1 and 4 random amino acids.

Animal experiments have shown that TKPR branched peptides (TKPR)₄>K₂>K-G prepared using the 4-copy branched peptide (X_{A}-X_{B}-X_{C}-X_{D}-X₁)₄>(K-X₂)₂>K-X₃ of the invention have strong activities for improving immunity and inhibiting tumor growth.

Animal experiments have shown that TKPR branched peptides (TKPR)₄>K₂>K-TKPR prepared using the 4-copy branched peptide (X_{A}-X_{B}-X_{C}-X_{D}-X₁)₄>(K-X₂)₂>K-X₃ of the invention have strong activity for improving immunity.

The 4-copy branched peptide of the invention, for example, (TKPR)₄>K₂>K-G or (TKPR)₄>K₂>K-TKPR, overcomes the disadvantage of the linear chain oligopeptide TKPR to be easily degraded in organisms, maintains immunological enhancement activity and anti-tumor effect of the TKPR fragment, and obviously improves biologic activities thereof. Pharmaceutical compositions comprising the 4-copy branched peptide are finally degraded in organisms into free amino acids, which can be directly absorbed without apparent drug residues and side effects. As drugs, the peptide has high security and development potential in clinical applications.

Advantages of the 4-copy branched peptide (X_{A}-X_{B}-X_{C}-X_{D}-X₁)₄> (K-X₂)₂>K-X₃ are summarized below:

1. The 4-copy molecule can be degraded into smaller oligopeptide fragments under the action of enzymes in organisms, and then functions in the form of a single copy molecule.

2. With regard to synthesis technology, the 4-copy branched peptide is prepared on a solid phase resin and, to a start-up carboxyl terminal (C terminal), one amino acid or a single chain oligopeptide comprising several amino acids, i.e., the branched peptide represented by the formula of (X_{A}-X_{B}-X_{C}-X_{D}-X₁)₄>(K-X₂)₂>K-X₃, are linked; the X₃ amino acid sequence functions as an "arm" and increases space between the branched peptide and the solid phase resin, thereby lowering space steric hindrance among branched peptide molecules compactly distributed on solid phase resin and improving synthetic efficiency.

3. Test experiments have shown that after between 1 and 4 amino acids introduced to the carboxyl terminal (C terminal) of the 4-copy branched peptide, the biological activities of the 4 branched active peptide are not shielded or decreased, on the contrary, they have been improved a lot compared with that of a pure 4-copy branched peptide.

The invention provides a 4-copy branched peptide represented by formula of (X_{A}-X_{B}-X_{C}-X_{D}-X₁)₄> (K-X₂)₂>K-X₃, 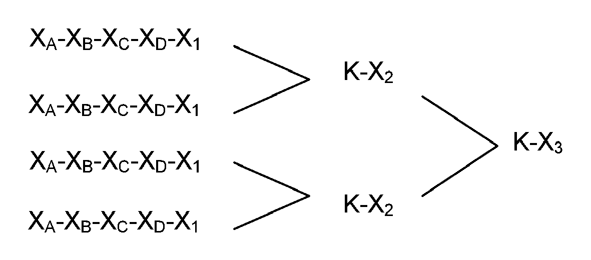 Specifically, the 4-copy branched peptide is (TKPR)₄>K₂>K-G. Experiments have shown that the polypeptide can improve immunity and anti-tumor activity. The structure of the polypeptide is:

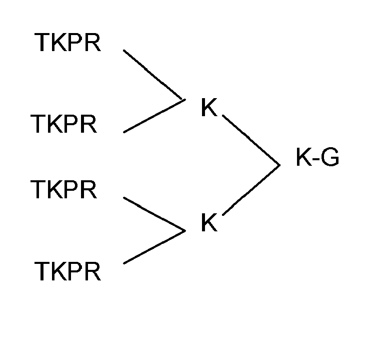

To further elaborate the technical scheme of the invention, the design mode, specific application, implementation mode, characteristics, and efficacy of the 4-copy branched peptide (TKPR)₄>K₂>K-G are provided below.

In 1963, American scientist R.B.Merrifield invented a method for synthesizing peptide chains using a solid phase. Specifically, the carboxyl terminal (C terminal) of the carboxyl terminal amino acid of a target peptide was fixed to an insoluble resin, and an addition condensation reaction was conducted between the amino group (N terminal) of the amino acid fixed to the resin and the carboxyl group of an amino acid to be introduced to prolong a peptide chain. The polypeptide synthesis was conducted using the condensation reaction from the carboxyl terminal (C terminal) to the amino terminal (N terminal) of the peptide chain. When the condensation reaction was conducting, the amino groups and side chain groups of the amino acids to be introduced should be protected. Conventional protection reagents include tertbutyloxy carbonyl (Boc) and fluorenylmethyloxy carbonyl (Fmoc). Therefore, for each condensation reaction, a de-protection reaction had to be conducted on the amino terminal of the peptide so as to make the amino group react with the activated carboxyl terminal of an amino acid to be introduced. Through such procedures, the reaction was conducted repeatedly, i.e., condensation - washing - de-protection - neutralization and washing - a next round condensation (introducing another amino acid), until a desired peptide chain was synthesized.

After the polypeptide synthesis reaction had been complete, the peptide was split from the resin using TFA or HF, and separated and purified using high performance liquid chromatography (HPLC) C18 reversed-phase chromatographic separation column. Based on the above principle, the polypeptide may be synthesized by manual operation, or by a polypeptide synthesizer through inputting synthesis sequence and automatic programs. At present, solid phase methods have been employed as a common technique to synthesize polypeptides and proteins.

It should be noted that the synthetic mode of the 4-copy TKPR branched peptide fragment is an amino acid condensation reaction conducted on two free active amino groups of lysine, and the obtained fragments are further synchronously prolonged using amino acid addition condensation reactions to yield a multiple copy branched peptide. In the process of synthesizing a linear chain polypeptide, only one amino group of lysine is activated, and the other is protected from a condensation reaction. In case a multiple copy branched peptide is required, two free amino groups of lysine (represented by >K-) are activated and separately condensed with two amino acids. If two lysine (>K-) are employed, then four amino acids can be introduced continuously. In this way, a multiple copy branched polypeptide molecule can be obtained after successive amino acid addition condensations.

Biological activities of polypeptide molecules are determined by amino acid sequence and structure thereof. Polypeptide synthesis has become a common technique and there are commercial service companies providing synthetic products required by clients. Here concrete details and principles of polypeptide synthesis and purification are not given again, please refer to "Fmoc Solid Phase Peptide Synthesis: A Practical Approach"; W. C. Chan (Editor), Peter D. White (Editor); Publisher: Oxford University Press, New York, USA; 1 Edition (March 2, 2000). The mode of synthesizing and preparing branched peptide of the invention can refer to the above solid phase synthesis mode but is not limited thereto.

### Example 1

Tuftsin, a 4-peptide, i.e., Thr-Lys-Pro-Arg (TKPR), is an active oligopeptide produced by spleen and has strong immune enhancement and anti-tumor activities. To prepare a polypeptide product with highly-efficient immune enhancement and anti-tumor activities, a 4-copy branched peptide (TKPR)₄>K₂>K-G comprising TKPR has been designed and prepared.

In the example, an ABI433A polypeptide solid phase synthesizer is employed. Raw materials involved comprise Fmoc-Thr (tBu), Fmoc-Lys (Boc), Fmoc-Pro, Fmoc-Avg (Pbf), Fmoc-Lys (Fmoc), and Fmoc-Gly. The solid phase resin is Wang resin (100-200 meshes). Polypeptide synthesis is conducted from carboxyl terminal (C terminal) to amino terminal (N terminal) using condensation reactions. Lys employed at the 2- and 4-branched position is Fmoc-Lys (Fmoc). Lys employed in the linear chain is Fmoc-Lys (Boc). After peptide chain synthesis has been completed, the protecting group Fmoc at the terminal is removed. The peptide chain is split from the resin using TFA/water. TFA is removed using vacuum distillation. The peptide chain is separated and purified using HPLC C18 reversed-phase column with water/ TFA/ acetonitrile as a mobile phase for gradient elution. The obtained polypeptide product is freeze dried to yield a white floccus solid.

In the embodiment, the polypeptide TKPR (with molecular weight of 500.6 Dalton) and the 4-copy branched peptide (TKPR)₄>K₂>K-G (with molecular weight of 2389.97 Dalton) are obtained using artificial solid phase synthesis. The synthetic products are separated and purified using HPLC C18 column with water/ TFA/acetonitrile as a mobile phase, with a final purity exceeding 98.0%.

Detection of anti-tumor activities of the branched peptide

1. Test objective:

Evaluation of the effect of the branched peptide (TKPR)₄>K₂>K-G on the growth of a transplanted tumor of Kunming mice with H22 liver cancer.

Experimental animal: Kunming mice, SPF class, 4-6 weeks old, 15-20 g, female, 10 mice in each group.

2. Experimental method and medication:

Under aseptic conditions, about 6 mL of ascites from two mice with H22 cancers was collected and diluted with aseptic normal saline by a ratio of 1:5. The oncocyte concentration was about 1.76×10⁷/mL. 0.2 mL of the ascites was subcutaneously injected to the right forefoot axilla of the mice. The inoculated cells were about 3.52×10⁶ per mouse. After inoculated with cancer cells, the mice were weighed, classified accordingly, and divided into groups randomly. After 24 hours of the tumor injection, the mice were administered with drugs.

Different groups were administered with different drugs as follows:

Blank control group: 200 µL normal saline each time;

Positive control group: 2 mg/kg of TKPR; and

Experimental group: 2 mg/kg of (TKPR)₄>K₂>K-G.

Each group of animals were intraperitoneally injected with drugs every other day, i.e., at the first day, the fifth day, the seventh day, the ninth day, and the eleventh day after the tumor implantation conducted. Every other day, the mice were weighed, the size of the tumor measured using a vernier calipers, and the volume of the tumor calculated accordingly. 12 days later, the animals were killed by neck dislocation. The tumor lump and spleen were collected and weighed, and on which based, the efficacy of the drugs was evaluated.

3. Results:

Results showed that (TKPR)₄>K₂>K-G had obvious effect on the growth of transplanted H22 liver tumor of Kunming mice. The calculated inhibition ratio based on the tumor weight was 48.4%, and the calculated inhibition ratio based on the tumor volume was 55.9%, both of which indicated (TKPR)₄>K₂>K-G significantly inhibited the growth of the tumor, exhibiting statistics significance (P<0.001).

In the test duration, the animals of the experimental groups didn't die, no acute toxicity reactions occurred, and activities of each group animals were normal. The body weight growth of the animals of the experimental groups was the same as that of the blank control group. The spleen weight of each group of animals had no obvious difference. The tested drug (TKPR)₄>K₂>K-G had no obvious side effect. Tables **1**, **2**, and **3** list the detail data of each group.

**Table 1 Effect of (TKPR)₄>K₂>K-G on growth of transplanted H22 liver cancer tumor of Kunming mice**

| Groups | Dosage (mg/kg) | Administration mode | Number of animals | | Average weight (X±S, g) | | Tumor weight (W) (g) | Tumor volume (V) (mm³) | Tumor inhibition ratio (%) | | Weight of spleen (g) |
|---|---|---|---|---|---|---|---|---|---|---|---|
| | | | Start | End | Start | End | | | W | V | |
| Blank control group | NS | ip×5 | 10 | 10 | 15.1± 0.8 | 29.2±1.2 | 3.14± 0.97 | 3693.2±645.8 | - | - | 0.35± 0.11 |
| Positive control group | 2 | ip×5 | 10 | 10 | 15.2± 0.7 | 29.6±1.7 | 2.43± 0.91* | 2830.9 ±952.5 | 22.6 | 23.4 | 0.32± 0.09 |
| Experimental group | 2 | ip×5 | 10 | 10 | 14.8± 0.8 | 30.6±3.2 | 1.62± 0.85** | 1628.3± 837.6*** | 48.4 | 55.9 | 0.31 ± 0.08 |

| | | | | | | | | | | | |
|---|---|---|---|---|---|---|---|---|---|---|---|
| **: P<0.01 vs Blank control group; ***: P<0.001 vs Blank control group. | | | | | | | | | | | |

**Table 2 Effect of (TKPR)₄>K₂>K-G on volume growth of transplanted H22 liver cancer tumor of Kunming mice**

| Groups | Tumor volume (mm³) | | | |
|---|---|---|---|---|
| | The 5th day after administration | The 7th day after administration | The 9th day after administration | The 11 th day after administration |
| Blank control group | 75.4±31.2 | 660.7±256.0 | 1920.3±406.6 | 3693.2±645.8 |
| Positive control group | 53.6±37.3 | 571.7±204.6 | 1623.7±483.4 | 2830.9±952.5 |
| Experimental group | 48.2±32.0 | 390.2±205.2* | 1251.6±500.3** | 1628.3±837.6*** |

| | | | | |
|---|---|---|---|---|
| *: P<0.05 vs Blank control group; **: P<0.01 vs Blank control group; ***: P<0.001 vs Blank control group. | | | | |

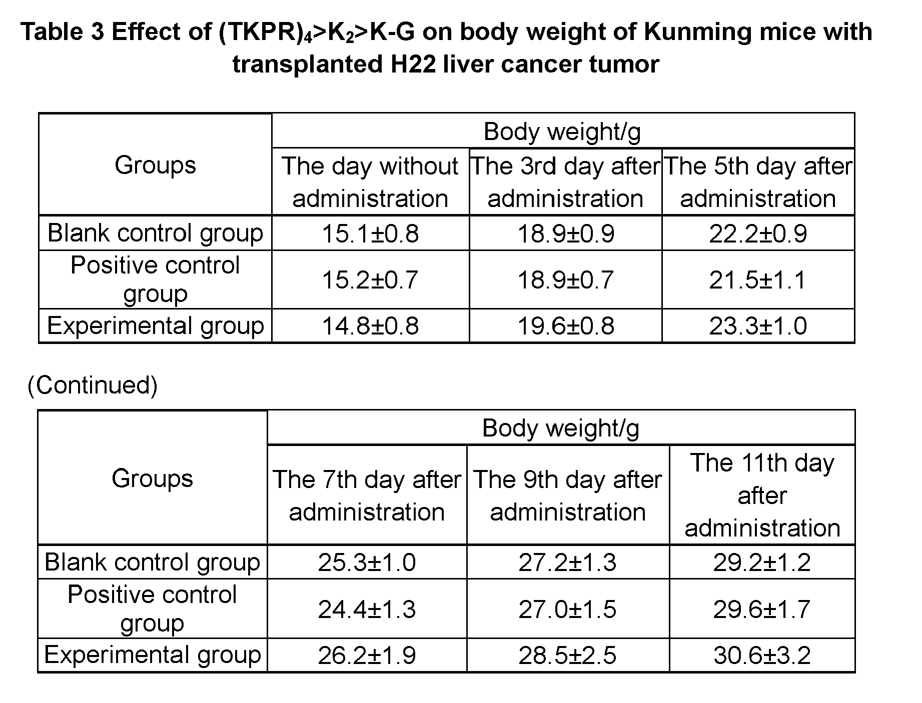

### Example 2

A 4-copy branched peptide represented by formula of (X_{A}-X_{B}-X_{C}-X_{D}-X₁)₄>(K-X₂)₂>K-X₄ was designed. An ABI433A polypeptide solid phase synthesizer is employed. Raw materials involved comprise Fmoc-Thr (tBu), Fmoc-Lys (Boc), Fmoc-Pro, Fmoc-Avg (Pbf), Fmoc-Lys (Fmoc), and Fmoc-Gly. The solid phase resin is Wang resin (100-200 meshes). Polypeptide synthesis is conducted from carboxyl terminal (C terminal) to amino terminal (N terminal) using condensation reactions. Lys employed at the 2- and 4-branched position is Fmoc-Lys (Fmoc). Lys employed in the linear chain is Fmoc-Lys (Boc). After peptide chain synthesis has been completed, the protecting group Fmoc at the terminal is removed. The peptide chain is split from the resin using TFA/water. TFA is removed using vacuum distillation. The peptide chain is separated and purified using HPLC C18 reversed-phase column with water/ TFA/ acetonitrile as a mobile phase for gradient elution. The obtained polypeptide product is freeze dried to yield a white floccus solid. The structure is as follows:

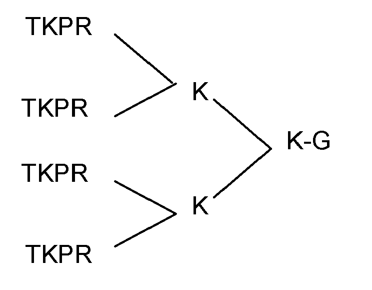

In the embodiment, the polypeptides TKPR (with molecular weight of 500.6 Dalton), (TKPR)₄>K₂>K (with molecular weight of 2332.92 Dalton), (TKPR)₄>K₂>K-G (with molecular weight of 2389.97 Dalton), and the 4-copy branched peptide (TKPR)₄>K₂>K-TKPR (with molecular weight of 2815.51 Dalton) are obtained using artificial solid phase synthesis. The synthetic products are separated and purified using HPLC C18 column with water/TFA/acetonitrile as a mobile phase, with a final purity exceeding 98.0%.

Experimental method:

Newcastle Disease Virus (NDV) can agglutinate with chicken red blood cell, which is a specific antibody neutralization reaction. The principle thereof is that the virus hemagglutinin is agglutinable with erythrocytes. If a specific antibody is first added to react with the virus, followed by addition of erythrocytes, no agglutination reaction occurs. The experiment is called as hemagglutination inhibition test (HI). The highest dilution ratio of antiserum applied to the test is a titer of the antibody. The higher the titer of an antibody, the better the immune effect is. HI method has following advantages: (1) high sensibility, capable of detecting trace amount of antibodies, with accurate result, being one of sensitive serological reactions; (2) high specificity, the agglutination reaction between virus and erythrocytes only being inhibited by a specific antibody; (3) high detection speed, a result being available in only about 2 hours; (4) low demand for environment, simple operation, and capability of detecting a large number of samples for one time. Therefore, the hemagglutination inhibition test (HI) has become a common detection method for detecting serum antibody of poultries. For more details, please refer to "Animal Immunology lab tutorials", chief editor: Guo Xin, China Agricultural University, 2007.

Test objective:

Inactivated vaccine of Newcastle Disease Virus is combined with different 4-copy branched peptide products, for example,(TKPR)₄>K₂>K-G, (TKPR)₄>K₂>K-TKPR, and (TKPR)₄>K₂>K to vaccinate SPF chicken and detect HI antibody, and observe whether the synthetic peptide products (TKPR)₄>K₂>K-G and (TKPR)₄>K₂>K-TKPR can enhance the immunity of chickens and compare the experimental results of the two peptide products with that of (TKPR)₄>K₂>K.

Materials and method:

SPF chickens about one month old were collected and divided into 5 groups, with each group 10 chickens. 0.3 mL of Newcastle disease inactivated vaccine (La Sota) was injected into breast muscle of the chickens.

Different groups were administered with different drugs as follows:

Blank group: 0.3 mL of normal saline was injected into breast muscle of each chicken.

Normal group: 0.3 mL of inactivated vaccine was injected into breast muscle of each chicken.

Experimental group 1: 0.3 mL of inactivated vaccine comprising 1 µg of (TKPR)₄>K₂>K was injected into breast muscle of each chicken.

Experimental group 2: 0.3 mL of inactivated vaccine comprising 1 µg of (TKPR)₄>K₂>K-G was injected into breast muscle of each chicken.

Experimental group 3: 0.3 mL of inactivated vaccine comprising 1 µg of (TKPR)₄>K₂> K-TKPR was injected into breast muscle of each chicken.

Feeding manner: Each group of chickens is fed separately.

Blood collection: Blood on the 28^{th} day after vaccination was collected and serum separated for HI tests.

Detection results show that the HI antibody titer of the experimental groups comprising synthetic products (TKPR)₄>K₂>K-G or (TKPR)₄>K₂>K-TKPR are significantly higher than that of the experimental group (TKPR)₄>K₂>K group. The results are listed in Table 4.

**Table 4 HI results on the 28^{th} day after the immunization**

| Serial number | 1 | 2 | 3 | 4 | 5 | 6 | 7 | 8 | 9 | 10 | Mean value |
|---|---|---|---|---|---|---|---|---|---|---|---|
| Blank group | 0 | 0 | 0 | 0 | 1 | 0 | 0 | 0 | 1 | 0 | <1 |
| Normal group | 3 | 3 | 4 | 3 | 3 | 2 | 4 | 3 | 4 | 3 | 3.2 |
| Experimental group 1 | 4 | 5 | 4 | 3 | 5 | 4 | 2 | 5 | 4 | 5 | 4.1 |
| Experimental group 2 | 3 | 5 | 3 | 4 | 6 | 4 | 4 | 6 | 5 | 7 | 4.7 |
| Experimental group 3 | 4 | 3 | 6 | 5 | 4 | 5 | 6 | 5 | 3 | 5 | 4.6 |

Result evaluations:

Test results show that the combination of 4-copy branched peptide (TKPR)₄>K₂>K-G or (TKPR)₄>K₂>K-TKPR with vaccine can significantly improve average titer of antibody of vaccine, and the improvement effect is better than that of the combination of (TKPR)₄>K₂>K with vaccine. Test animals had no abnormity or side effect. Thus, compared with (TKPR)₄>K₂>K, 4-copy branched peptides (TKPR)₄>K₂>K-G and (TKPR)₄>K₂>K-TKPR have stronger immunostimulation effect.

Summary:

The products (TKPR)₄>K₂>K-G and (TKPR)₄>K₂>K-TKPR are prepared in accordance with the formula of (X_{A}-X_{B}-X_{C}-X_{D}-X₁)4>(K-X₂)₂>K-X₃ of the invention, in which -X₃ at the carboxyl terminal (C terminal) of the branched peptide is substituted respectively with the amino acid G and TKPR, wherein X₃ represents between 1 and 5 random amino acids. Experiments have shown that, the connection of an amino acid or short peptide to the carboxyl terminal of the 4-copy branched peptide (1) does not eliminate functions of single copy oligopeptide TKPR, and original biologic activities of the single copy oligopeptide TKPR are retained; (2) significantly improves biological activities of a pure 4-copy branched peptide (TKPR)₄>K₂>K. Therefore, it is proved that peptide molecules designed and prepared in accordance with the formula (X_{A}-X_{B}-X_{C}-X_{D}-X₁)₄>(K-X₂)₂>K-X₃ have more strong immunity enhancement and anti-tumor activities. They can be independently used as raw materials of drugs or be prepared into drugs to inhibit tumor growth or improve immunity in clinical applications.

The 4-copy branched peptide can be modified as follows to yield a series of derivatives thereof to inhibit tumor growth or improve immunity in clinical applications:

1. Hydroxyl groups of the 4-copy branched peptide can form but it is not limited to ethers, esters, glycosides, glucosides, etc.;

2. Sulfydryl of the 4-copy branched peptide can form but it is not limited to a thioether, thioglycoside, or compound containing disulfide bond formed by the sulfydryl and cysteine or a peptide containing cysteine;

3. Amino groups of the 4-copy branched peptide can form but it is not limited to acyl compounds, alkylation compounds, glycoside substance formed by the amino groups with carbohydrate substance, etc.;

4. Carboxyl groups of the 4-copy branched peptide can form but it is not limited to esters, amide compounds, etc.;

5. Imino groups of the 4-copy branched peptide can form but it is not limited to glycosides, acyl compounds, alkylation compounds, etc.;

6. Phenolic hydroxyl groups of the 4-copy branched peptide can form but it is not limited to esters, ethers, glycosides, glucosides, and salt compounds formed by the phenolic hydroxyl with an organic base or inorganic base;

7. Salt compounds formed by the 4-copy branched peptide with an organic acid or inorganic acid;

8. Complexes, clathrate, or chelate formed by the 4-copy branched peptide with a metal ion; and

9. Hydrates or solvents of the 4-copy branched peptide.

Industrial application:

The 4-copy branched peptide of the invention, for example, (TKPR)₄>K₂>K-G, (TKPR)₄>K₂>K-TPRR, or a derivative thereof prepared according to any one or more of above-mentioned chemical modifications, can be made into anti-tumor drugs to inhibit tumor growth and enhance immunity in clinical applications.

## Claims

1. A 4-copy branched peptide, **characterized in that**, the peptide having a formula of (X_{A}-X_{B}-X_{C}-X_{D}-X₁)4>(K-X₂)₂>K-X₃,
wherein
X_{A} and X_{c} separately represent an uncharged polar amino acid;
X_{B} and X_{D} separately represent an alkaline amino acid;
K represents lysine generally with only one free activated amino group for amino acid addition condensation reactions, and >K-represents lysine comprising two free activated amino groups for amino acid addition condensation reactions;
X₁ and X₂ separately represents an amino acid sequence comprising between 0 and 5 random amino acids, and X₁ and X₂ are the same, different, or absent; and
X₃ is a sequence comprising between 1 and 4 random amino acids.

2. The 4-copy branched peptide of claim 1, **characterized in that** the uncharged polar amino acid is selected from the group consisting of serine (Ser, S), threonine (Thr, T), cysteine (Cys, C), praline (Pro, P), glutamine (Gln, Q), and asparagine (Asn, N).

3. The 4-copy branched peptide of claim 1, **characterized in that** the alkaline amino acid is selected from the group consisting of histidine (His, H), lysine (Lys, K), and arginine (Arg, R).

4. The 4-copy branched peptide of claim 1, **characterized in that** X_{A} is Thr, X_{B} is Lys, X_{C} is Pro, X_{D} is Arg, X₁ and X₂ are zero, X₃ is glycine (Gly, G), and the peptide is (TKPR)₄>K₂>K-G.

5. The 4-copy branched peptide of claim 1, **characterized in that** X_{A} is Thr, X_{B} is Lys, X_{C} is Pro, X_{D} is Arg, X₁ and X₂ are zero, X₃ is Thr-Lys-Pro-Arg, and the peptide is (TKPR)₄>K₂>K-TKPR.

6. The 4-copy branched peptide of any of claims 1-5, **characterized in that** a hydroxyl group of the 4-copy branched peptide forms an ether, an ester, a glycoside, or a glucoside, but not limited thereto.

7. The 4-copy branched peptide of any of claims 1-5, **characterized in that** a sulfydryl of the 4-copy branched peptide forms a thioether, a thioglycoside, or a compound containing a disulfide bond formed by the sulfydryl with cysteine or a peptide containing cysteine, but not limited thereto.

8. The 4-copy branched peptide of any of claims 1-5, **characterized in that** an amino group of the 4-copy branched peptide forms an acyl compound, an alkylation compound, or a glycoside substance formed by the amino group with a carbohydrate substance, but not limited thereto.

9. The 4-copy branched peptide of any of claims 1-5, **characterized in that** a carboxyl group of the 4-copy branched peptide forms an ester or an amide compound, but not limited thereto.

10. The 4-copy branched peptide of any of claims 1-5, **characterized in that** an imino group of the 4-copy branched peptide forms a glycoside, an acyl compound, or an alkyl compound, but not limited thereto.

11. The 4-copy branched peptide of any of claims 1-5, **characterized in that** a phenolic hydroxyl group of the 4-copy branched peptide forms an ester, ether, glycoside, glucoside, or salt compound formed by the phenolic hydroxyl group with an organic base or inorganic base, but not limited thereto.

12. The 4-copy branched peptide of any of claims 1-5, **characterized in that** the 4-copy branched peptide reacts with an organic base or inorganic base to yield a salt compound.

13. The 4-copy branched peptide of any of claims 1-5, **characterized in that** the 4-copy branched peptide reacts with a metal ion to yield a complexes, clathrate, or chelate.

14. The 4-copy branched peptide of any of claims 1-5, **characterized in that** the 4-copy branched peptide is a hydrate or solvent thereof.

15. A pharmaceutical composition for inhibition of tumor growth comprising a 4-copy branched peptide of any of claims 1-14 or a derivative thereof.

16. A pharmaceutical composition for enhancement of immunity comprising a 4-copy branched peptide of any of claims 1-14 or a derivative thereof.
